**Europäisches Patentamt**

**European Patent Office**

**Office européen des brevets**

(19)

(11) Veröffentlichungsnummer: **0 288 944 B1**

# (12) EUROPÄISCHE PATENTSCHRIFT

(45) Veröffentlichungstag der Patentschrift: **03.07.91**

(51) Int. Cl.5: **A61F 5/01**

(21) Anmeldenummer: **88106559.3**

(22) Anmeldetag: **23.04.88**

(54) **Flexible Spreizlagerungs-Bandage zur Behebung von Hüftdefekten bei Säuglingen.**

(30) Priorität: **28.04.87 DE 3714083**

(43) Veröffentlichungstag der Anmeldung:
**02.11.88 Patentblatt 88/44**

(45) Bekanntmachung des Hinweises auf die
Patenterteilung:
**03.07.91 Patentblatt 91/27**

(84) Benannte Vertragsstaaten:
**AT BE CH DE ES FR GB GR IT LI NL SE**

(56) Entgegenhaltungen:
**DE-A- 2 018 240**
**DE-A- 3 508 844**
**DE-U- 8 226 590**

(73) Patentinhaber: **S + G ORTHOPÄDIETECHNIK GMBH**
**Grapengiesserstrasse 34**
**W-2400 Lübeck(DE)**

(72) Erfinder: **Prinz, Edgar**
**Waschgrabenstrasse 7**
**W-2430 Neustadt(DE)**
Erfinder: **Dufek, Pavel**
**Percevalstrasse 5**
**W-2400 Lübeck(DE)**
Erfinder: **Grundei, Hans**
**Hamburger Strasse 89**
**W-2400 Lübeck(DE)**

(74) Vertreter: **Wilcken, Thomas, Dipl.-Ing. et al**
**Musterbahn 1**
**W-2400 Lübeck(DE)**

EP 0 288 944 B1

**Beschreibung**

Die Erfindung geht aus von einer flexiblen Spreizlagerungs-Bandage zur Behebung von Hüftdefekten bei Säuglingen wie sie in der DE-A-20 18 240 offenbart ist. Bei dieser bekannten Spreizlagerungs-Bandage ist ein als feste Schiene ausgebildeter, vorn offener Gürtel durch Verschlußbänder verschließbar. Der Gürtel ist beidseitig mit Beinhaltern durch einstellbare Stäbe und Gurtschleifen verbunden. Diese bekannten Bandagen haben keine stabile Lage am Körper des Säuglings, da sich die den Gürtel bildende Schiene leicht verschieben kann.

Es ist weiter eine Spreizlagerungs-Bandage für den gleichen Zweck aus einem Prospekt der Firma BASKO Orthopädie-Versand GmbH & Co. KG, betreffend die "Pavlik-Bandage" bekannt, bei der ein schmaler Gurt durch Schulterbänder gehalten wird und durch in der Länge einstellbare Bandschleifen mit Fußformteilen verbunden ist. Auch diese Bandagen besitzen keine eindeutige stabile Lage am Körper eines Säuglinges und die Fußformteile sind nur bedingt einstellbar.

Die Aufgabe der Erfindung besteht darin, bei SpreizlagerungsBandagen der eingangs erwähnten Art eine zuverlässige, stabile Lage der beiden Hüften von Säuglingen zu erreichen, wobei die Beinhalter nach jeder Beinbewegung des Säuglinges automatisch in die eingestellte Lage zurückbewegt werden, so daß auch dann die stabile Lage der Hüften beibehalten wird, und wobei Verletzungen für die unteren Extremitäten vermieden werden.

Diese Aufgabe wird durch die Merkmale des Patentanspruches 1 gelöst.

Durch diese Lösung wird dem Gürtel eine zuverlässige, stabile, sich nicht verschiebende Stellung am Säugling erteilt und damit wird auch durch die seitlichen dorsalwärtigen flexiblen Kunststoffstäbe eine zuverlässige, sich stets wieder einstellende stabile Flexions-und Abduktionsstellung der beiden Hüften erreicht, aber es wird die erforderliche Beweglichkeit des Säuglinges beibehalten.

In allen Fällen sind alle Teile der Bandage an unterschiedliche Säuglingsgrößen anpaßbar und austauschbar, und auch eine Größenanpassung an wachsende Säuglinge ist möglich.

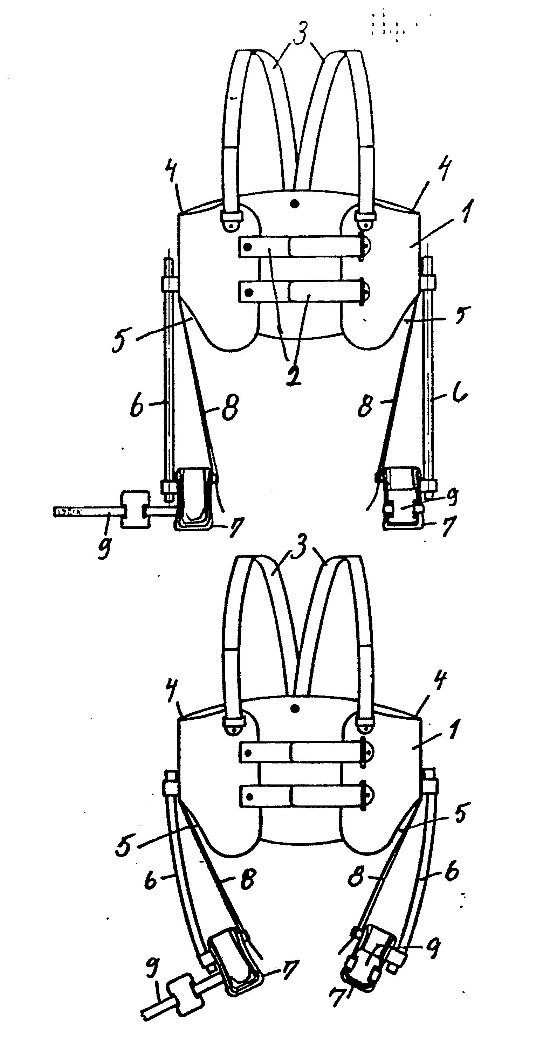

Die Erfindung wird nachstehend anhand der Zeichnung erläutert, in der die Spreizlagerungs-Bandage für Säuglinge in Vorderansicht mit zwei verschiedenen Stellungen der elastischen, vom Gürtel zu den Fußformteilen verlaufenden Stäbe gezeigt ist.

Die flexible Spreizlagerungs-Bandage zur Behebung von Hüftleiden bzw. Hüftdefekten von Säuglingen besteht aus einem um den Leib des Säuglinges herumzulegenden Gurt in Form einer festen, elastischen,vorn offenen Kunststoffschiene 1. Die offene Vorderseite ist durch Verschlußbänder 2 bzw. einen Klettenverschluß verschließbar und dadurch ist die Kunststoffschiene 1 in der Weite einstellbar und an die Größe eines Säuglinges anpaßbar. Der als Gürtel dienende Teil 1 wird durch Schulterbänder 3 und durch beidseitige, obere Kantenausnehmungen 4 für die Achselhöhlen und untere Ausnehmungen 5 für die Leistenbeugen in einer stabilen Lage gehalten.

Der Gürtel 1 ist beidseitig nach dorsalwärts mit elastischen Kunststoffstäben fest verbunden, die anderenends außen mit Fußformteilen 7 aus festem Kunststoff mit weicher Innenauflage verbunden sind. Die Länge und die Winkelstellung der elastischen Stäbe 6 können im Bereich des Gürtels 1 oder der Fußformteile 7 eingestellt werden. Weiter greift an dem Gürtel 1 seitlich nach ventralwärts je eine elastische, in der Länge einstellbare Gurtschleife 8 an, die ebenfalls, und zwar medial, mit den Fußformteilen 7 verbunden sind. Die beiden Fußformteile 7 sind vorn durch Klettenverschluß 9 verschließbar.

Die einzeln beschriebenen Teile der Spreizlagerungs-Bandage sind an unterschiedliche Säuglingsgrößen anpaßbar und auch austauschbar. Die Bandage ist einfach für Eltern und Pflegepersonal zu handhaben. Die Bandage erzielt eine zuverlässige, stabile Flexions- und Abduktionsstellung beider Hüften und bildet damit eine wirksame Bandage zur Behebung und Heilung angeborener Hüftdefekte bei Säuglingen.

**Ansprüche**

1. Flexible Spreizlagerungs-Bandage zur Behebung von Hüftdefekten bei Säuglingen, bestehend aus einem in der Weite einstellbaren, als feste Schiene (1) ausgebildeten, vorn offenen und über Verschlußbänder (2) verschließbaren Gürtel, an den auf beiden Seiten dorsalwärts Stäbe (6) im gewünschten Winkel einstellbar befestigt sind und seitlich ventralwärts je eine elastische Gurtschleife (8) zwischen Gürtel (1) und Fußformteil (7) angebracht sind, dadurch gekennzeichnet, daß die durch an sich bekannte Schulterbänder (3) gehaltene feste Schiene (1) mit geschwungenen Kantenausnehmungen (4) für die Achselhöhlen und unteren Ausnehmungen (5) für die Leistenbeugen versehen ist und daß die an ihm befestigten Stäbe (6) aus flexiblen Kunststoff bestehen.

2. Bandage nach Anspruch 1, dadurch gekennzeichnet, daß die beiden flexiblen Kunststoffstäbe (6) gegenüber den Fußformteilen (7) und die beiden Gurtschleifen (8) in der Länge einstellbar sind.

3. Bandage nach Anspruch 1 oder 2, dadurch gekennzeichnet, daß die Fußformteile (7) aus einem festen Kunststoff, mit einer weichen Kunststoffeinlage versehen, vorn durch Klettenverschluß verschließbar sind, und daß an den medialen Seiten der Fußformteile die elastischen Gurtschleifen (8) und an der Außenseite die elastischen Stäbe (6) einstellbar befestigt sind.

**Claims**

1. Flexible straddle positioning truss for correcting hip defects in infants, consisting of a girdle which is adjustable in width, constructed as a fixed splint (1), open at the front and closable by closure bands (2) and to which are attached on both sides towards the dorsal region rods (6) adjustable to the desired angle and at each side towards the ventral region an elastic belt loop (8) between girdle (1) and foot shape portion (7), characterised in that the fixed splint (1), which is held by shoulder straps (3) known in the art, is provided with curved edge openings (4) for the armpits and lower openings (5) for the groin curvatures and that the rods (6) attached to it are made of flexible plastics material.

2. Truss according to claim 1, characterised in that the two flexible plastic rods (6) are adjustable relative to the foot shape portions (7) and the two belt loops (8) are adjustable in length.

3. Truss according to claim 1 or 2, characterised in that the foot shape portions (7) made of a rigid plastics material, provided with a soft plastic inlay, are closable at the front by burr-type fastening, and that the elastic belt loops (8) are adjustably attached to the medial sides of the foot shape portions and the elastic rods (6) are adjustably attached to the outside.

**Revendications**

1. Bandage flexible de mise en position écartée, pour le traitement de défauts de la hanche chez les nourrissons, constitué d'une ceinture réglable en largeur, en forme d'attelle rigide (1), ouverte à l'avant et pouvant être fermée à l'aide de bandes de fermeture (2) , sur laquelle des barreaux (6) sont fixés dans le dos sur les deux côtés selon un angle souhaité réglable et, sur chaque côté, une boucle élastique de ceinture (8) est disposée sur le ventre entre la ceinture (1) et la partie de forme de pied (7), caractérisé en ce que l' attelle rigide (1) , maintenue par des bandes d'épaules (3) connues en soi, est pourvue d'évidements (4) à arêtes incurvées pour les aisselles et d'évidements inférieurs (5) pour les plis de l'aine et en ce que les barreaux (6) qui lui sont fixés sont en matière synthétique flexible.

2. Bandage selon la revendication 1, caractérisé en ce que les deux barreaux flexibles (6) en matière synthétique sont réglables par rapport aux parties de forme de pied (7) et en ce que les deux boucles de ceinture (8) sont réglables en longueur.

3. Bandage selon la revendication 1 ou 2, caractérisé en ce que les parties de forme de pied (7) en matière synthétique rigide , pourvues d'une garniture en matière plastique souple, peuvent être fermées sur le devant par un ruban auto-accrochant, et en ce que les boucles élastiques de ceinture (8) et les barreaux élastiques (6) sont fixés de façon réglable, respectivement, aux côtés médians et au côté extérieur des parties de forme de pied.